# EUROPEAN PATENT SPECIFICATION

(11) **EP 0 920 630 B1**
(45) Date of publication and mention of the grant of the patent: **31.07.2002**
(21) Application number: 97927319.0
(22) Date of filing: 29.05.1997
(51) Int. Cl.: G01N 33/68, C12Q 1/68, C12N 15/62, A61K 38/17

(54) **PRION BINDING PROTEINS AND USES THEREOF**
PRIONBINDENDE PROTEINE UND DEREN VERWENDUNGEN
PROTEINES DE LIAISON AU PRION ET LEUR UTILISATION

(30) Priority: 29.05.1996 US 18380 P
(43) Date of publication of application: 09.06.1999
(60) Divisional application: 01124615.4
(73) Proprietor: McGILL UNIVERSITY, Montreal, Quebec H3A 2A7 (CA)
(72) Inventor: CASHMAN, Neil, R., Toronto, Ontario M5S 3H2 (CA); DODELET, Vincent, Montreal, Quebec H2T 2T5 (CA)
(74) Representative: Vossius, Volker, Dr.
(86) International application number: IB9700827
(87) International publication number: WO9745746

(56) References cited:
- WO-A-92/08713
- WO-A-97/16728
- PRIOLA SA ET AL: "Prion protein and the scrapie agent: in vitro studies in infected neuroblastoma cells." INFECT AGENTS DIS, APR-JUN 1994, 3 (2-3) P54-8, UNITED STATES, XP002056072
- OESCH, BRUNO ET AL: "Identification of cellular proteins binding to the scrapie prion protein" BIOCHEMISTRY (1990), 29(24), 5848-55 CODEN: BICHAW;ISSN: 0006-2960, XP002056073
- FLANAGAN JG ET AL: "The kit ligand: a cell surface molecule altered in steel mutant fibroblasts." CELL, OCT 5 1990, 63 (1) P185-94, UNITED STATES, XP002056074
- WEISS S ET AL: "OVEREXPRESSION OF ACTIVE SYRIAN GOLDEN HAMSTER PRION PROTEIN PRPC AS A GLUTATHIONE S-TRANSFERASE FUSION IN HETEROLOGOUS SYSTEMS" JOURNAL OF VIROLOGY, vol. 69, no. 8, August 1995, pages 4776-4783, XP000644456
- TELLING G C ET AL: "PRION PROPAGATION IN MICE EXPRESSING HUMAN AND CHIMERIC PRP TRANSGENES IMPLICATES THE INTERACTION OF CELLULAR PRP WITH ANOTHER PROTEIN" CELL, vol. 83, no. 1, 6 October 1995, pages 79-90, XP000674450
- KURSCHNER, CORNELIA ET AL: "Analysis of interaction sites in homo- and heteromeric complexes containing Bcl-2 family members and the cellular prion protein" MOL. BRAIN RES. (1996), 37(1,2), 249-58 CODEN: MBREE4;ISSN: 0169-328X, XP002056075
- CAUGHEY, BYRON ET AL: "Binding of the protease-sensitive form of prion protein PrP to sulfated glycosaminoglycan and Congo red" J. VIROL. (1994), 68(4), 2135-41 CODEN: JOVIAM;ISSN: 0022-538X, XP002056076
- OESCH, BRUNO: "Characterization of PrP binding proteins" PHILOS. TRANS. R. SOC. LONDON, SER. B (1994), 343(1306), 443-5 CODEN: PTRBAE;ISSN: 0080-4622, 1994, XP002041123
- CHEMICAL ABSTRACTS, vol. 122, no. 5, 30 January 1995 Columbus, Ohio, US; abstract no. 49358, OESCH, BRUNO ET AL: "Interaction of the prion protein with cellular proteins" XP002056077 & PRION DIS. HUM. ANIM. (1992), 398-406. EDITOR(S): PRUSINER, STANLEY B. PUBLISHER: HORWOOD, LONDON, UK. CODEN: 60BWAD,
- YEHIELY, FRUMA ET AL: "Identification of candidate proteins binding to prion protein" NEUROBIOL. DIS. (1997), 3(4), 339-355 CODEN: NUDIEM;ISSN: 0969-9961, 1997, XP002041124
- TIG, vol.10(8), pages 286-291, (1994)

### ANNEX TO PATENT

### SEQUENCE LISTING

(1) GENERAL INFORMATION:
   (i) APPLICANT:
      (A) NAME: MCGILL UNIVERSITY
         Office of Technology Transfer
      (B) STREET: 3550 University Street
      (C) CITY: Montreal
      (D) STATE: Quebec
      (E) COUNTRY: Canada
      (F) POSTAL CODE (ZIP): H3A 2A7
   (ii) TITLE OF INVENTION: PRION PROTEIN BINDING PROTEINS AND USES THEREOF
   (iii) NUMBER OF SEQUENCES: 6
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA:
      (A) APPLICATION NUMBER: PCT/IB97/00827
      (B) FILING DATE: 29-MAY-1997
   (vi) PRIOR APPLICATION DATA:
      (A) APPLICATION NUMBER: US 60/018,380
      (B) FILING DATE: 29-MAY-1996
(2) INFORMATION FOR SEQ ID NO:1:
   (i) SEQUENCE CHARACTERISTICS:
      (A) LENGTH: 549 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:1:
   GCTTCCACCA TCATTCACTA TGAGATCCTG GAGGAGAGAG AGAGGGGGTT CCCCGTGGGT   60
   AACGTGGTCA CGGATCTTGG TTTGGATCTC GGCAGTCTGT CGGCCCGCCG GCTCCGGGTG  120
   GTGTCCGGAG CTAGCCGTAG GTTCTTTGAG GTGAACTGGG AGACTGGAGA GATGTTCGTC  180
   AACGATCGAC TGGACCGAGA GGAGCTGTGC GGGACGCTGC CCTCCTGCAC TGTAACTCTG  240
   GAGTTGGTGG TAGAGAACCC GCTGGAGCTG TTCAGCGCGG AAGTGGTGGT CCAGGACATC  300
   AACGACAACA ATCCCTCTTT CCCCACCGGG GAAATGAAAT TGGAGATTAG CGAGGCCTTG  360
   GCGCCGGGGA CGCGCTTTCC GCTCGAGAGC GCGCACGATC CCGATGTGGG GAGCAACTCT  420
   TTACAAACCT ATGAGCTGAG CCACAATGAG TACTTTGCGC TCCGCGTGCA GACTCGAGAA  480
   GACGGCACGA AATATGCGGA GCTGGTCCTG GAGCGCGCCC TAGATTGGGA ACGGGAGCGC  540
   CGTCTTCAG                                                          549
(2) INFORMATION FOR SEQ ID NO:2:
   (i) SEQUENCE CHARACTERISTICS:
      (A) LENGTH: 183 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:2:
   Ala Ser Thr Ile Ile Glu Tyr Glu Ile Leu Glu Glu Arg Glu Arg Gly
    1               5                  10                  15
   Phe Pro Val Gly Asn Val Val Thr Asp Leu Gly Leu Asp Leu Gly Ser
               20                  25                  30
   Leu Ser Ala Arg Arg Leu Arg Val Val Ser Gly Ala Ser Arg Arg Phe
           35                  40                  45
   Phe Glu Val Asn Trp Glu Thr Gly Glu Met Phe Val Asn Asp Arg Leu
       50                  55                  60
   Asp Arg Glu Glu Leu Cys Gly Thr Leu Pro Ser Cys Thr Val Thr Leu
   65                  70                  75                  80
   Glu Leu Val Val Glu Asn Pro Leu Glu Leu Phe Ser Ala Glu Val Val
                   85                  90                  95
   Val Gln Asp Ile Asn Asp Asn Asn Pro Ser Phe Pro Thr Gly Glu Met
               100                 105                 110
   Lys Leu Glu Ile Ser Glu Ala Leu Ala Pro Gly Thr Arg Phe Pro Leu
           115                 120                 125
   Glu Ser Ala His Asp Pro Ile Val Gly Ser Asn Ser Leu Gln Thr Tyr
       130                 135                 140
   Glu Leu Ser His Asn Glu Tyr Phe Ala Leu Arg Val Gln Thr Arg Glu
   145                 150                 155                 160
   Asp Gly Thr Lys Tyr Ala Glu Leu Val Leu Glu Arg Ala Leu Asp Trp
                   165                 170                 175
   Glu Arg Glu Arg Arg Leu Gln
               180
(2) INFORMATION FOR SEQ ID NO:3:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: Other
   (xi) SEQUENCE DESCRIPTION: SEQ ID NO:3:
   AGACATAAGC TTGCAGCCAT CATGGCGAAC CTTGGC                                 36
(2) INFORMATION FOR SEQ ID NO:4:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: Other
   (xi) SEQUENCE DESCRIPTION: SEQ ID NO:4:
   GAGATTGGAT CCTCTTCTCC CGTCGTAATA G                31
(2) INFORMATION FOR SEQ ID NO:5:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: Other
   (xi) SEQUENCE DESCRIPTION: SEQ ID NO:5:
   AGACATAAGC TTGCAGCCAT CATGGCGAAC CTTGGC           36
(2) INFORMATION FOR SEQ ID NO:6:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: Other
   (xi) SEQUENCE DESCRIPTION: SEQ ID NO:6:
   GAGATTGGAT CCTCTCTGGT AATAGGCCTG                  30

## Description

### Background of the Invention

This invention relates to prion protein binding proteins, nucleic acids, their identification and uses thereof.

The prion diseases are a group of rapidly progressive, fatal, and untreatable neurodegenerative syndromes. The human prion disorders include Creutzfeldt-Jakob disease (CJD), which can be transmitted through accidental contamination of parenteral therapeutic agents (such as pituitary hormones extracted from cadavers) and transplanted tissues (such as corneas and dural grafts). To date, the Canadian Red Cross has withdrawn over 12 million dollars of blood products from the Canadian market because of potential contamination from donors who have subsequently developed CJD, or who have had family members with the disease. In addition, scrapie in sheep and goats is a common and economically important prion-related disease in North America, as is bovine spongiform encephalopathy in Great Britain. The latter also has major health and economic implications for human consumption of beef and preparation of biological products from this species.

The prion diseases are characterized pathologically by spongiform change (i.e., microcavitation of brain, usually predominant in gray matter), neuronal cell loss, astrocytic proliferation out of proportion to neuronal loss, and accumulation of an abnormal amyloidogenic protein, sometimes in discrete plaques in the brain. It is possible that neurodegeneration in prion diseases shares certain underlying mechanisms with other more common neurodegenerative syndromes such as Alzheimer's disease and amyotrophic lateral sclerosis and Parkinson's disease.

The agents which transmit these diseases differ markedly from viruses and viroids in that no chemical or physical evidence for a nucleic acid component has been reproducibly detected in infectious materials (Prusiner, Science 216:136-144, 1982). A purification method for the scrapie agent that utilized proteinase K digestion led to the discovery of a 27-30 kD protease-resistant protein in scrapie-affected hamster brain, termed PrP27-30 (Bolton et al., Science 218:1309-1311, 1982). PrP27-30 co-purified with scrapie agent activity and subsequently was shown to be the major (or only) macromolecule of the transmissible agent (McKinley et al., Cell 35:57-62, 1983). PrP27-30 was later determined to be a proteolytic digestion product of a 33-37 kD complete form of the scrapie agent protein (termed PrP^{sc}), which is also capable of disease transmission.

When a partial amino acid sequence for PrP27-30 was determined and cDNAs were cloned, the gene coding for this protein was found to be host-derived (Oesch et al., Cell 40:735-746, 1985). This cellular protein has been isolated from normal brain and, unlike PrP^{sc}, it is protease-sensitive and not associated with scrapie disease-producing activity (Bendheim et al., Ciba Found. Symp. 164-177, 1988). It is hypothesized that infectious particle-associated PrP^{sc} is derived from a normal cellular precursor, PrP^{c} (Prusiner, Science 252:1515-1522, 1991). Recently, cell-free PrP^{sc}-catalyzed conversion of PrP^{c} to PrP^{sc} has been reported (Kocisko et al., Nature 370:471-473, 1994). The protease-sensitive normal cellular isoform, PrP^{c}, is an evolutionarily conserved membrane protein of unknown function. Recently, PrP^{c}, which is a glycosyl-phosphatidylinositol (GPI)-linked protein, was shown to modify T cell activation induced by concanavalin A stimulation (Cashman et al., Cell 61:185-182, 1990), suggesting an important functional role for this protein.

Transmission of prion diseases between species is limited by a "species barrier" predominantly determined by PrP amino acid sequences. Moreover, recent transgenic experiments also suggest a role for a species-specific macromolecule distinct from PrP which also participates in prion agent formation. Transgenic mice expressing high levels of human chimeric PrP^{C}, inoculated with brain extracts from humans with prion disease, were resistant to human prions. Susceptibility to human prions occurred only upon ablation of the mouse PrP gene, or expression of a human-mouse chimeric prion gene. These findings suggest the possible existence of species-specific binding protein with higher affinity to mouse than human prion proteins (Telling et al., Cell 83:79-90, 1995).

Prion protein *in vitro* interacts with Congo red and certain sulfated polysaccharides (Priola, S.A. et aL, Infect. Agents Dis. 3:54-58, 1994). Furthermore, Caughey et al. disclose interactions between prion protein and both Congo red and heparin (Caughey, B. et aL, J. ViroL 68:2135-2141, 1994). Three Pli proteins have been shown to bind non-specifically to prion protein under *in vitro* conditions (Oesch, B. et al., Biochemistry 29:5848-5855, 1990; Oesch, B., Phil. Trans. R. Soc. London, Ser. B, 343:443-445, 1994) and an interaction between prion protein and Bc1-2 fusion protein under non-physiological conditions has been disclosed (Kurschner, C. and Morgan, J.I., MoL Brain Res. 37:249-258, 1996).
A prion protein fusion to glutathione S-transferase has been disclosed (Weiss, S. et al., J. ViroL 69:4776-4783, 1995). A general teaching regarding alkaline phosphatase fusion proteins is described by Flanagan and Leder (Flanagan, J.G. and Leder, P., Cell 63:185-194, 1990). WO 92/08731 describes substantially purified T-cadherin polypeptides and nucleic acids coding for said T-cadherin polypeptides. Furthermore, a method for inhibiting tumor cell migration by increasing cell adhesion with an effective amount of T-cadherin is disclosed.

There are currently no sensitive diagnostic tests on the market and no therapeutic treatments for human prion diseases.

### Summary of the Invention

In general, the invention isolates and uses substantially pure prion protein binding proteins (PrPBPs). These proteins are characterized as binding in a saturable and displacable manner to a prion protein (PrP). Preferably, the binding is also of high affinity, and results in the generation of appropriate functional signals (for example, inhibition of cellular proliferation or enhanced levels of cell death). Two PrPBPs are described below; these proteins, which are isolated from murine cells, are cell surface proteins. The cloning and characterization of the genes encoding these PrPBPs is described herein. Other preferable PrPBPs may be isolated from cell or tissue samples derived from any organism, although mammals represent preferred sources and, in particular, humans and any domesticated farm animal or pet species.

Also disclosed are purified nucleic acids which encode PrPBPs; vectors and cells containing those nucleic acids; antibodies which bind specifically to PrPBPs; and methods of producing a recombinant PrPBP involving providing a cell transformed with DNA encoding a PrPBP positioned for expression in the cell, culturing the transformed cell under conditions for expressing the DNA, and recovering the recombinant PrPBP.

Such PrPBPs are useful for the detection and treatment of prion diseases, e.g., degenerative syndromes such as the muscular dystrophies and disorders involving abnormal cell proliferation or abnormal cell death. These PrPBPs are also useful for the decontamination of samples known or suspected to contain prion proteins.

In another aspect, a fusion protein having a PrP portion and an alkaline phosphatase portion is disclosed. Preferably, this protein leaves intact the binding and functional capabilities of the PrP domain. Such PrP-AP fusion proteins provide useful affinity reagents for the labelling, detection, or identification of PrPBPs or PrP^{sc}'s in a cell, tissue, fluid, or other biological sample.

In addition, PrP-AP fusion proteins facilitate the identification and affinity purification of PrPBP proteins in cell samples, and also facilitate the cloning of PrPBP coding sequences. In one particular method according to the invention, frog oocytes, which express minimal background surface PrPBPs are injected with *in vitro* transcribed mRNA from a cDNA library pool or clone, contacted with a PrP-AP fusion protein, and cells expressing a protein to which PrP-AP binds are identified thus allowing unabigious identification of cDNA clones encoding PrPBP. By purifying the cDNA from those cells, the PrPBP-coding sequence may be readily recovered.

In a related aspect, methods and kits for detecting PrP^{sc} in a biological sample are disclosed. The method may include the steps of: (a) destroying PrP^{c} in the biological sample (for example, by proteolytic degradation); (b) contacting the biological sample with a PrPBP; and (c) detecting complex formation between the PrPBP and PrP^{sc} of the biological sample. The detection of such complex formation is taken as indicating the presence of PrP^{sc} in the biological sample. The PrPBP used in the detection assay may be a fusion protein (e.g., PrPBP-AP) or a PrPBP fragment or analog that binds to PrP^{sc}. In a preferred embodiment, complex formation is detected using PrPBP (or a fragment or analog thereof) that is directly labeled. In another embodiment, complex formation is detected indirectly, e.g., by using an antibody directed against PrPBP (or a fragment or analog thereof). In still another embodiment, the detection of PrP^{sc} is determined by measuring the displacement of labeled PrP^{sc} in a PrPBP:PrP^{sc} complex with unlabelled PrP^{sc} present in the biological sample.

In another related aspect, the invention features methods for decontaminating PrP^{sc} from a biological sample. In a preferred embodiment, the method involves the steps of: (a) treating the biological sample with PrPBP which is encoded by a nucleic acid molecule identifiable by the method of claim 1 for a sufficient period of time, the treatment permitting the formation of PrPBP:PrP^{sc} complex formation; and (b) recovering the PrPBP:PrP^{sc} complex from the biological sample. Such a decontamination method may also involve the use of perfusing a biological sample (e.g., cells or organs prepared for transplantation) with PrPBP (or a fragment or analog thereof) for the removal or inactivation of PrP^{sc}.

In another embodiment, PrP^{sc} bound to PrPBP (or fragment thereof) may be detected by anti-PrP antibodies.

PrPBPs which binds PrP^{sc} can be used to quatitate PrP^{sc} in diagnostic or other assays.

If a given PrPBP does not bind to PrP^{sc} and instead binds only to PrP^{c}, the PrPBP can be used to remove PrP^{c} from a sample containing both PrP^{sc} and PrP^{c}. Then an antibody which binds both PrP^{sc} PFP^{c} can be used to detect PrP^{sc} remaining in the sample.

Further, a method of treating or preventing a prion disease in an animal (for example, a human) is disclosed. Preferred, the method involves administering to the animal a therapeutically effective amount of a compound that antagonizes PrPBP:PrP^{sc} complex formation, blocks conversion of PrP^{C} to PrP^{sc}, or inhibits a PrPBP:PrP^{sc} complex-mediated biological activity. In another embodiment, the method involves administering to the animal a therapeutically effective amount of a compound that antagonizes the conversion of PrP^{c} to PrP^{sc}.

A method of suppressing a PrPBP:PrP^{sc} complex-mediated biological activity in an animal is also disclosed. Preferred, the method involves administering to the animal a compound (e.g., an antibody directed against PrPBP, PrPBP, or a fragment or analog thereof) that inhibits PrPBP:PrP^{sc} complex interaction. In addition, treatment with PrPBPs may block the generation of cytotoxic signals within the body by preventing the interaction of PrP^{c} with PrP^{sc}.

In another aspect, the invention features a method of identifying a compound for the ability to decrease a binding interaction between PrPBP (or a fragment or analog thereof) and PrP. In a preferred embodiment, the method involves the steps of: (a) mixing a compound with a PrPBP and a PrP; (b) measuring binding of the PrPBP to the PrP in the presence of the compound; and (c) identifying whether the compound decreases binding of the PrPBP to the PrP relative to a control sample.

Further, another therapeutic method of treating neurodegenerative syndromes by suppressing functional activation PrPBP. Preferred, the method involves administering to the animal a compound; e.g., small molecule antagonist or agonist, peptide, or a peptidomimetic which blocks or enhances the generation of intracellular signals from PrPBPs.

Certain diseases, e.g., neurodegenerative diseases, can be treated by administering a molecule which increases or decreases the interaction between a cadherin which is a PcPBP and PrP^{c}. For example, all or a PcP binding portion of a cadherin can be used to interfere with the interaction of endogenous cadherin and PrP^{c}.

In addition compounds useful for the treatment of neurodegenerative diseases, can be identified by determining whether a selected test compound increases or decreases the binding between a cadherin and PcP^{c}.

By "prion protein binding protein" or "PrPBP" is meant any protein which binds to a "prion protein" or "PrP" in a saturable and displaceable manner. Preferably, this binding is also "high affinity" under normal physiological conditions and conformation-dependent. PrPBPs are preferably present on the cell surface during at least a portion of their normal cellular existence, but may also be naturally present (or engineered to be present) all or part of the time in the cytoplasm, cytoplasmic organelles, or nucleus of a host cell. PrPBPs may also be present as a family of proteins in a particular cell, tissue, or organism, and PrPBPs from any organism (and, in particular, from mammals such as humans or domesticated animals, for example, sheep, cows, cats, and goats) are included in the definition.

By "high affinity" binding is meant an affinity constant (between a prion protein and PrPBP) of less than 100 µM, less than 10 µM, less than 1 µM, less than 100 nM, preferably less than 10 nM, and more preferably less than 2 nM or even 1 nM.

By "saturable" binding is meant binding (between a prion protein and PrPBP) which stops increasing after having reached a certain maximal level, indicating that there are a finite number of binding sites for one of the proteins, and that these binding sites are specific. This is in contrast to the non-specific and continually increasing binding which is characteristic of a protein which adheres non-specifically to cell surfaces.

By "conformation dependent binding" is meant binding that occurs on a non-denatured protein that has been properly post-translationally modified, folded and transported such that its normal physiological binding characteristics remain intact.

By "competitive" binding is meant binding (between a prion protein and PrPBP) which is progressively inhibited by increasing concentrations of an unlabeled form of one of the proteins (for example, PrP).

By "prion diseases" is meant a group of rapidly progressive, fatal, and untreatable brain degenerative disorders including, without limitation, Creutzfeldt-Jakob disease (CJD), Kuru, Gerstmann-Straussler syndrome, and fatal familial insomnia in humans (Prusiner, Science 252:1515-1522, 1991), scrapie in sheep and goats, and spongiform encephalopathy in cattle, as well as recently described prion disease in other ruminants and cats.

By "treatment of prion diseases" is meant the ability to reduce, prevent, or retard the onset of any symptom associated with prion diseases, particularly those resulting in spongiform change, neuronal cell loss, astrocytic proliferation, accumulation of PrP^{sc} protein, dementia, and death.

By "substantially pure" is meant a preparation which is at least 60% by weight (dry weight) the compound of interest. Preferably, the preparation is at least 75%, more preferably at least 90%, and most preferably at least 99%, by weight the compound of interest. Purity can be measured by any appropriate method, e.g., polyacrylamide gel electrophoresis or HPLC analysis.

By "purified DNA" is meant DNA that is not immediately contiguous with both of the coding sequences with which it is immediately contiguous (one on the 5' end and one on the 3' end) in the naturally occurring genome of the organism from which it is derived. The term therefore includes, for example, a recombinant DNA which is incorporated into a vector; into an autonomously replicating plasmid or virus; or into the genomic DNA of a prokaryote or eukaryote, or which exists as a separate molecule (e.g., a cDNA or a genomic DNA fragment produced by PCR or restriction endonuclease treatment) independent of other sequences. It also includes a recombinant DNA which is part of a hybrid gene encoding additional polypeptide sequence.

By "transformed cell" is meant a cell into which (or into an ancestor of which) has been introduced, by means of recombinant DNA techniques, a DNA molecule encoding (as used herein) a PrPBP.

By "positioned for expression" is meant that the DNA molecule is positioned adjacent to a DNA sequence which directs transcription and translation of the sequence (i.e., facilitates the production of, e.g., a PrPBP).

By "purified antibody" is meant antibody which is at least 60%, by weight, free from the proteins and naturally-occurring organic molecules with which it is naturally associated. Preferably, the preparation is at least 75%, more preferably at least 90%, and most preferably at least 99%, by weight, antibody, e.g., PrPBP-specific antibody. A purified PrPBP antibody may be obtained, for example, by affinity chromatography using recombinantly-produced PrPBP and standard techniques.

By "specifically binds" is meant an antibody which recognizes and binds PrPBP but which does not substantially recognize and bind other molecules in a sample, e.g., a biological sample, which naturally includes PrPBP.

By "alkaline phosphatase" is meant any portion of an alkaline phosphatase protein which is capable of generating a detectable signal.

As noted above, the present application discloses two very important advances for the diagnosis and treatment of prion diseases. First, PrP fusion proteins which may be used to identify and isolate PrPBPs, and to clone the genes or cDNAs encoding these PrPBPs. In particular, one preferred PrP fusion protein makes use of an alkaline phosphatase fusion. This PrP-AP protein has numerous advantages. For example the PrP-AP protein has the advantage of minimally affecting the normal configuration of PrP, which may be necessary for binding (and, particularly, high affinity binding) to PrPBPs. Also, the PrP-AP fusion retains the ability to trigger normal cellular PrP functions, a significant advantage over prior PrP expression constructs. Perhaps most importantly, the PrP-AP fusion protein is capable of binding specifically and with high affinity to PrPBPs. Accordingly, this fusion protein provides a useful affinity reagent to sequester or isolate PrPBPs from tissue sources. This is advantageous because it provides a means for identifying new PrPBPs and also permits rapid purification of these proteins (for example, as new protein bands on gels) for protein microsequencing as well as for cloning of the cDNA or genomic sequences. In one particular technique, a PrP-AP fusion protein may be used for the identification and cloning of new PrPBPs by screening frog oocytes microinjected with *in vitro* transcribed mRNA from a cDNA library and selecting for clones which exhibit specific binding to the fusion protein. This method of PrPBP screening is advantageous because it permits the identification of PrPBPs which are normally expressed at very low levels in cells or which may not normally be accessible to PrP-AP binding. In addition, since the PrP-AP fusion protein includes alkaline phosphatase, an easily measurable enzyme, this fusion protein provides a useful tool for the quantitation and visualization of PrPBPs under experimental or diagnostic conditions.

In another important aspect, the invention provides, for the first time, prion protein binding proteins which bind in a saturable and displaceable manner to prion proteins. Such PrPBPs have numerous advantages. First, because these proteins bind specifically to PrPs, they may be labeled with detectable markers to provide an easy means to assay for the presence of PrP^{sc} in cells, tissues, or biological fluids. Also, when labeled, these proteins may be used in drug screening protocols (for example, competitive binding assays) for the identification of other pharmacological agents which bind to PrPs. Also, because of their specific and saturable binding affinity to PrP^{sc}, PrPBPs provide good candidates for the treatment of infected cells, tissues, or biological fluids by either removing PrP^{sc} through secondary separation techniques or by neutralizing the recruitment activity of PrP^{sc} in the body. PrPBPs are also advantageous in that they may be used to treat patients afflicted with prion diseases. For example, PrPBPs, peptide fragments, or analogues thereof, when injected into the body, may be used to prevent the conversion of PrP^{c} to PrP^{sc}, by binding to either of the prion proteins.

Other features and advantages of the invention will be apparent from the detailed description, and from the claims.

### Description of the Drawings

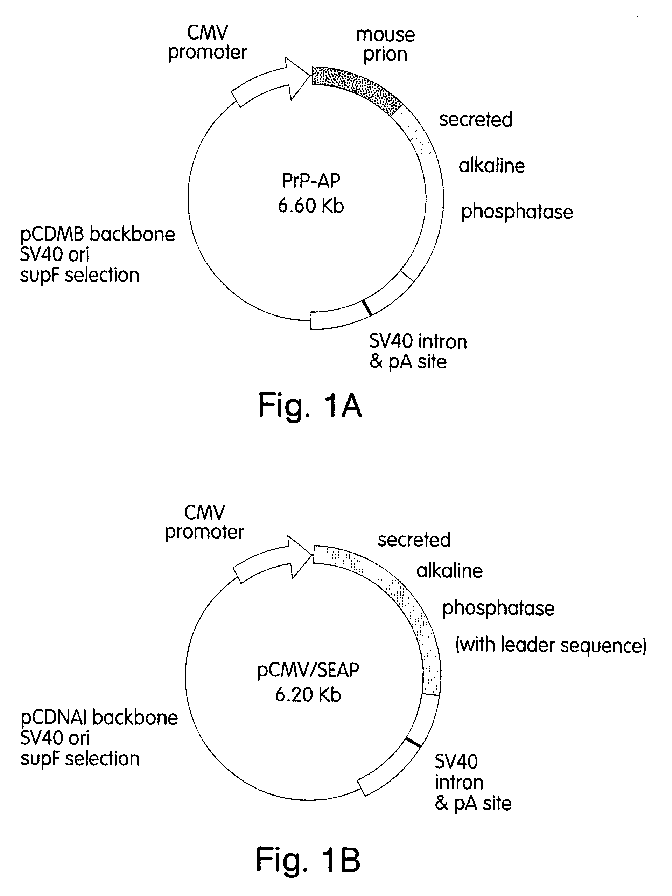

FIGURE 1A is a schematic representation of a murine PrP-AP fusion protein expression vector.

FIGURE 1B is a schematic representation of a control plasmid which expresses secreted alkaline phosphatase.

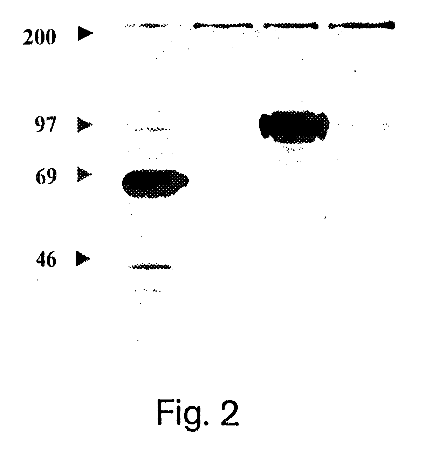

FIGURE 2 is a photographic representation of an autoradiograph illustrating that the PrP-AP fusion protein contains immunological determinants of both PrP^{c} and AP. ³⁵[S]-methionine labeled COS cell supernatants transfected with the CMV/SEAP vector expressing AP (lanes 1 and 2) or the APtag vector expressing PrP-AP (lanes 3 and 4) were immunoprecipitated with anti-AP monoclonal antibody (lanes 1 and 3) or PrP rabbit polyclonal antibody directed against the N-terminal 17 amino acids of the mature protein. Equivalent activities of AP were added to each immunoprecipitation reaction.

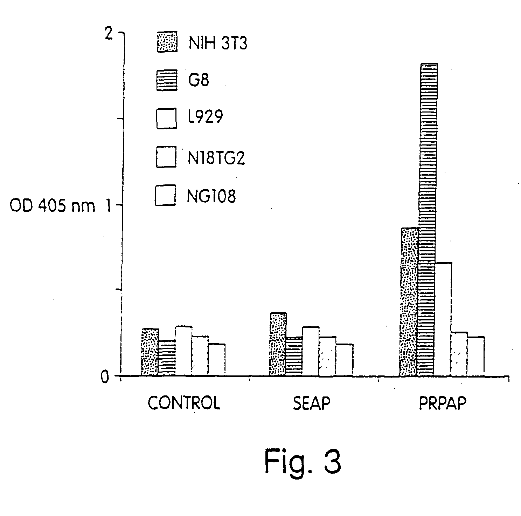

FIGURE 3 is a bar graph illustrating that PrP-AP adheres specifically to selected cell lines through the PrP^{c} domain. Three different mouse cell lines were cultured to confluency in 60 mm plates (approximately 100,000 cells) and were incubated with 3 ml COS cell test supernatants at room temperature for 90 minutes, washed, lysed, and alkaline phosphatase activity determined as described below. Control: conditioned media from mock-transfected COS cells containing no detectable alkaline phosphatase activity. SEAP: supernatant from COS cells transfected with the CMV/SEAP construct, displaying no surface binding compared to control. PrP-AP: supernatants from COS cells transfected with the PrP-AP construct, displaying surface binding. SEAP and PrP-AP supernatants contained equal alkaline phosphatase activity (approximately 500 OD units per hour).

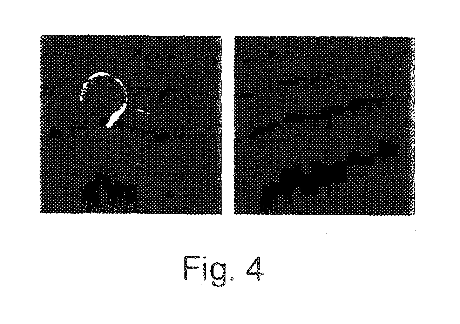

FIGURE 4 is a series of two photographic panels illustrating that PrP-AP, but not AP, recognizes a cell surface molecule by an "indirect immunofluorescence" protocol. G8 cells (a mouse muscle cell line) were mechanically dissociated without proteases, incubated with the PrP-AP or AP proteins, washed, incubated with an anti-AP monoclonal antibody, washed, incubated with goat anti-mouse immunoglobulin tagged with fluorescein isothiocyanate, washed, and examined by fluorescence microscopy. A fluorescent signal in PrP-AP-incubated cells (left panel) compared to AP-incubated cells (right panel) indicates specific cell surface binding mediated through the PrP domain of the PrP-AP fusion protein.

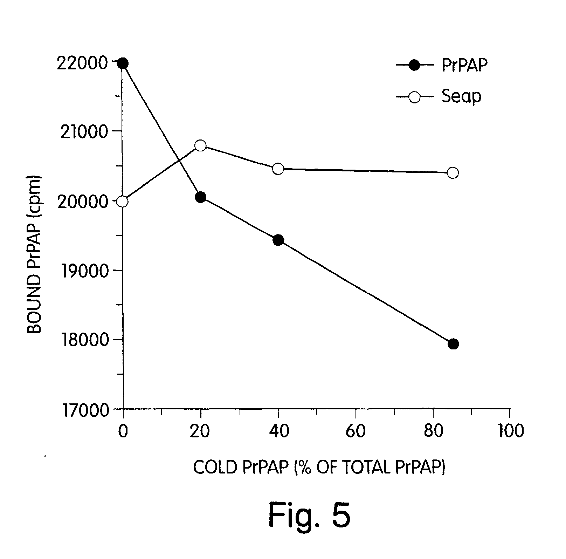

FIGURE 5 is a graph illustrating that PrP-AP binding is competitive and displaceable through its PrP^{c} domain. NIH 3T3 cells in 35 mm plates were incubated as above with supernatants from COS cells transfected with the PrP-AP construct which had been metabolically labeled with ³⁵[S]-methionine. Cells were subsequently washed, lysed, and bound radioactivity was quantified by β-counting. Bound ³⁵[S]-PrP-AP is progressively competed by increasing concentrations of "cold" unlabeled PrP-AP, but not by similar concentrations of AP from CMV/SEAP-transfected COS cells.

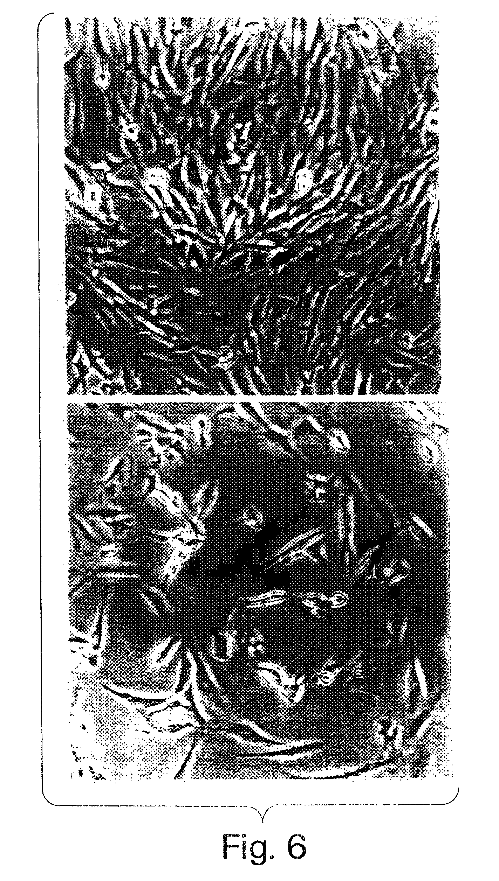

FIGURE 6 is a series of two photographic panels illustrating a decreased cell number in G8 muscle cell line cultures exposed to PrP-AP. Sister G8 cultures in 24 well plates were incubated for three days in COS cell supernatants containing AP at 25% v/v, or COS cell supernatants containing PrP-AP 25% v/v. Cell cultures exposed to PrP-AP were consistently less dense than AP-exposed cultures, suggesting that the PrPBP binding affects cell growth and/or viability.

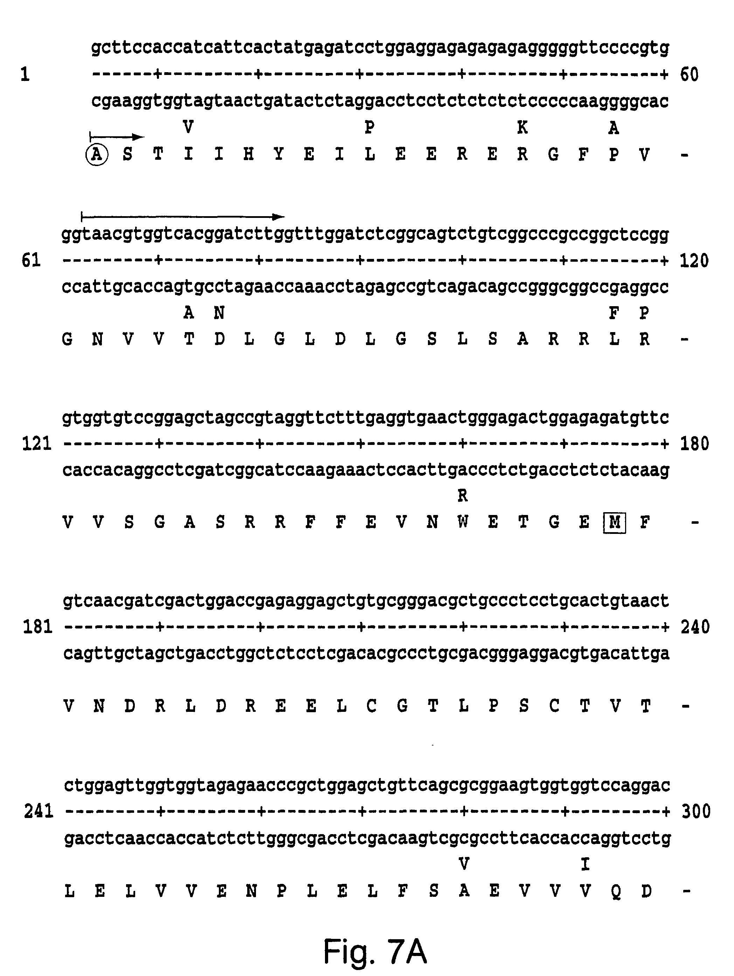

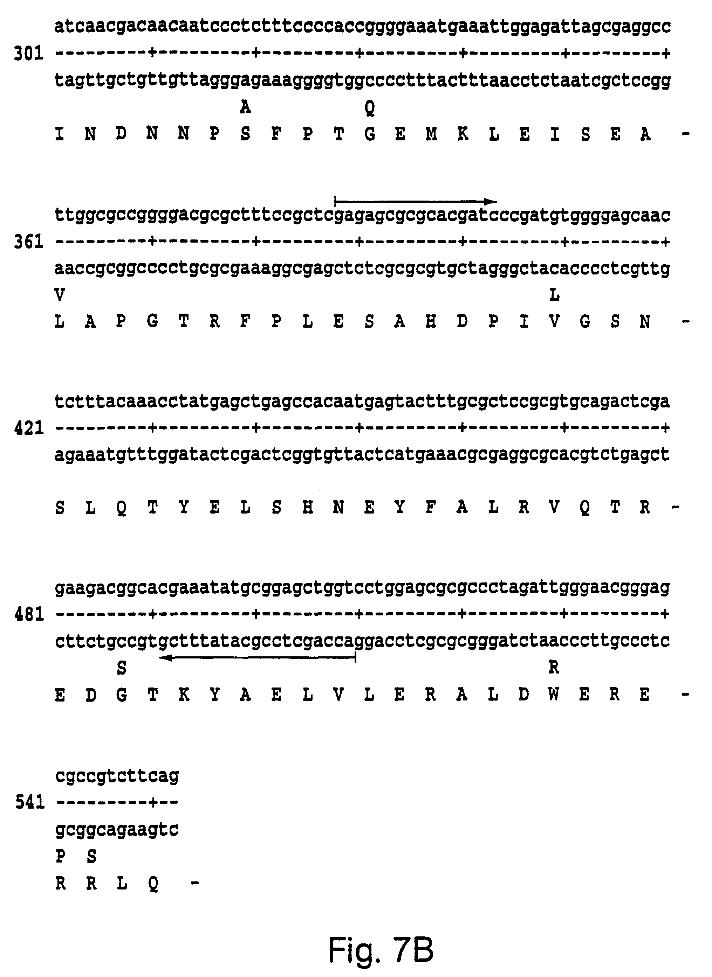

FIGURES 7A and 7B are the nucleic acid sequence and deduced amino acid sequence of the PrPBP of clone 6.

### Detailed Description

A PrP-AP fusion protein was constructed to serve as a marker and "affinity reagent" for PrPBPs. Novel PrPBPs were detected on the surfaces of select mouse cell lines. These PrPBPs bound to the PrP portion of the PrP-AP fusion protein with high affinity, in a competitive, saturable, and conformation-dependent fashion.

### Development of a PrP binding protein detection system

To develop a PrPBP detection system, a soluble recombinant fusion protein comprised of prion protein and alkaline phosphatase portions (designated PrP-AP) was constructed from a murine PrP gene in frame with secreted human placental heat-stable AP (Figure 1A). The murine PrP fragment (Locht et al., PNAS 83:6372-6, 1986; GenBank Accession #M13685) was generated by standard PCR from mouse brain cDNA and included the entire open reading frame from the initiating methionine (thus containing the PrP leader sequence) to Arg²²⁹ at the beginning of the GPI anchor attachment signal sequence (thus obviating the attachment of the GPI anchor). The PCR primers used to amplify this sequence are shown below: and These primers were designed to contain appropriate restriction sites (forward: HindIII, reverse: BamHI) for subsequent ligation into a HindIII-BglII site of the APtag-2 expression vector (Cheng and Flanagan, Cell 79:157-168, 1994), which contains the AP gene downstream from the cassette insert site. When the PCR product is ligated into this vector, the BamHI-BglII junction generates a Gly-Ser-Ser-Gly linker between the prion portion and the alkaline phosphatase portion of the fusion protein. The APtag-2 expression vector contains an SV40 origin of replication and a CMV promoter, allowing for vector amplification and high level fusion protein production in COS cells. In order to control for possible detection of proteins binding solely to the AP portion of the fusion protein, AP with its own leader sequence was expressed from the pCDNA1 vector (Invitrogen, San Diego, CA), also containing a CMV promotor and SV40 origin, and was designated CMV/SEAP (Figure 1B).

### The PrP-AP fusion protein contains immunological determinants of both PrP^{c} and AP

COS cells were transfected by standard DEAE-Dextran techniques with either the PrP-AP fusion protein vector or the CMV/SEAP vector. As expected, PrP-AP-transfected COS cell supernatants (Figure 2, lanes 3 and 4) incubated with ³⁵[S]-methionine and immunoprecipitated according to standard techniques (Antibodies, A Laboratory Manual, Harlow & Lane, Cold Spring Harbor Laboratory, Cold Spring Harbor, NY, 1988) with an AP monoclonal antibody (MIA 1801, human placenta; Medix Biotech, San Carlos, CA; lane 3) exhibited a prominent band at 97 kD, consistent with the presence of AP and the design of the PrP-AP vector. Immunoprecipitation of the same supernatants with a PrP rabbit polyclonal antibody (Bendheim et al., Nature 310:418-21, 1984; lane 4) also yielded a 97 kD band, indicating that PrP-AP-transfected COS cells secreted a 97 kD protein which was immunoreactive with both AP and PrP antibodies. Also as expected, CMV/SEAP-transfected COS cell supernatants (Figure 2, lanes 1 and 2) incubated with ³⁵[S]-methionine and immunoprecipitated with an anti-AP antibody as described above (lane 1) expressed a 67 kD protein, a molecular weight consistent with the design of the fusion protein and indicating the presence of AP in this protein. When these same COS cell supernatants were immunoprecipitated with polyclonal antibodies to PrP, no 67 kD protein was detected, indicating the absence of a prion moiety within the CMV/SEAP protein.

Both PrP-AP and AP were expressed and secreted by transfected COS cells at a concentration of about 5 µg protein per ml of supernatant, and both displayed similar supernatant AP activity (measured as described in Cheng and Flanagan, Cell 79:157-168, 1994). Supernatants of untransfected and mock-transfected COS cells did not contain detectable AP activity or immunodetectable PrP^{c}. This data demonstrated the successful construction and expression of the PrP-AP "affinity reagent" which was used to determine the distribution, function, and molecular identity of the PrP binding proteins.

### Detection of PrPBPs on the surface of select cell lines

To determine whether the PrP-AP protein was capable of detecting PrPBPs on cells, several different cell lines including some derived from mouse, human, primate, and neuronal/neuroblastoma cell lines, were cultured with DMEM/10% fetal calf serum (FCS) to confluence in 60 mm plates (approximately 100,000 cells). These cells were washed with phosphate buffered saline (PBS) and were incubated with 3 ml of COS cell supernatants containing the PrP-AP fusion protein or the CMV/SEAP protein (i.e., lacking the PrP moiety) in DMEM/5% FCS or conditioned media from mock transfected COS cells, at room temperature for 75-90 minutes. PrP-AP-transfected cell supernatants expressed on average 500 OD units/hr of AP activity (Cheng and Flanagan, supra), indicating the presence of about 5 µg/ml of PrP-AP fusion protein. Cells were subsequently washed, lysed, heated at 65°C for 10 minutes to inactivate endogenous phosphatase, and alkaline phosphatase activity determined as described above. In particular, AP activity was determined by conversion of p-nitrophenyl phosphate to a yellow product as measured by an ELISA reader (EAR 400 AT Easy Reader, SLT Instruments, Austria) at 405 nm.

Results obtained from 3 different mouse cell lines are illustrated in Figure 3. NIH 3T3 and L929 cells are derived from embryonic fibroblasts. G8 cells are myoblastoid (muscle lineage).

Also positive are human neuroblastoma lines SK-N-SH and SK-N-MC and glioma cell lines U87 and U373, human endothelial kidney (HEK) cells, primate COS cells, mouse and human peripheral lymphocytes, and mouse and human dissociated brain cells. PrPBPs are virtually ubiquitous and do not appear display species restricted binding. They are however, lacking from peripheral erythrocytes and *Xenopus* oocytes.

High AP activities were detected in heat-treated of G8, NIH 3T3, and L929 cell lines when incubated with the PrP-AP fusion protein derived from the supernatant of PrP-AP-transfected COS cells, (see "prpap" in Figure 3). This result indicates that PrPBPs are abundantly present on the surface of G8, NIH 3T3, and L929 cell types and are capable of binding PrP derived from the PrP-AP fusion protein with high affinity. Little or no AP activity was detected when these mouse cell lines were incubated with conditioned media from mock-transfected COS cell supernatants (i.e., in the absence of the PrP-AP fusion protein; see "control" in Figure 3). Also, little or no AP activity was detected when these cell lines were incubated with supernatants from CMV/SEAP-transfected COS cells, indicating that the detection of increased AP activity in G8, NIH 3T3, and L929 cells lines was due to the detection of specific PrPBP and not to other proteins binding only to the AP moiety of the PrP-AP fusion protein. In these experiments, SEAP and PrP-AP supernatants contained equal alkaline phosphatase activity (~500 OD units per hour). The fact that muscle cells expressed the high levels of PrPBP is consistent with their selective vulnerability in transgenic animals overexpressing PrP^{c} (Westaway et al., Cell 76:117-129, 1994).

To detect PrPBPs using a different technique and to determine their cellular localization, cells in suspension were labeled with the PrP-AP fusion protein using an indirect immunofluorescence assay. Cells were mechanically dissociated without proteases, triturated in Hank's Buffered Salt solution (HBSS), incubated with the PrP-AP or AP proteins for 60 minutes at room temperature, washed three times in ice-cold HBSS, and then incubated with anti-AP monoclonal antibodies (MIA 1801, human placenta, Medix Biotech, San Carlos, CA) for 30 minutes on ice. Cells were thereafter maintained at 4°C, washed again, and incubated with goat anti-mouse IgG coupled to fluorescein isothiocyanate (Jackson Immunoresearch, Philadelphia, PA), and washed and examined by fluorescence microscopy (on an Orthoplan fluorescence microscope) or flow cytometry (Facscan; Becton Dickinson, Oakville, Ontario, Canada).

Fluorescence microscopic results for G8 cells (mouse muscle cell line) are illustrated in Figure 4. A clear labeling of the cell surface was detected in G8 cells incubated with PrP-AP protein (Figure 4, left panel), but not with the AP protein alone (Figure 4, right panel). These results indicated that specific mouse PrPBPs exist on the cell surface of G8 cells. Other cell lines, including cos cells, and NIH 3T3 and L929 cells, exhibited similar results to those described for G8.

To identify the portion of the PrP which is binding to the PrPBPs on the surface of G8 and other cell lines, experiments equivalent to those described above, are performed, but in the presence of blocking domain antibodies which are specific for selected domains of the PrP protein. Particular blocking domain antibodies useful for this purpose include, for example, antibodies specific for the PrP carboxy terminus (hypothesized to determine species-specific binding by a "protein X"; Telling et al., Cell 83:79-80, 1995), antibodies specific for the middle codon region 96-176 (hypothesized to be critical in the recognition-conversion of PrP^{c} to PrP^{sc} and the apoptotic properties of PrP^{c} peptide; Forloni et al., Nature 362:543-546), and antibodies specific for the amino-terminal octapeptide repeat region (which figures prominently in familial CJD; Prusiner, Ann. Rev. Microbiol. 48:655-686, 1994). The structural features and amino acid sequence of the binding domain is then exploited to develop other compounds which bind to PrPBP with high affinity.

### Characterization of PrPBP binding

To determine whether PrPBPs bound PrP in a competitive manner, binding of ³⁵[S]-labeled PrP-AP was assessed in cell lines having previously shown PrP-AP binding activity. Cells grown to confluence in 35 mm culture dishes were incubated as described previously with the supernatants of COS cells transfected with the PrP-AP fusion protein which had been metabolically labeled with ³⁵[S]-methionine. The cells were subsequently washed, lysed, and bound radioactivity quantified by β-counting.

Results for NIH 3T3 cells are illustrated in Figure 5. Binding to PrPBPs by ³⁵[S]-labeled PrP-AP was inhibited by incubation with increasing concentrations of unlabeled PrP-AP, demonstrating competitive displacement of the labeled PrP by the unlabeled PrP. Furthermore, binding to PrPBPs (by ³⁵[S]-labeled PrP-AP) was not inhibited by increasing concentrations of AP derived from the CMV/SEAP fusion protein, indicating that binding was occurring through the PrP moiety, and not the AP moiety, of the fusion protein.

To determine the binding affinity between PrPBPs and the PrP as well as the total number of binding sites present on given cell populations, Scatchard analyses were performed according to a modification of the technique of Cheng and Flanagan (Cell 79:157-168, 1994) using G8 cells. The supernatants of COS cells transfected with PrP-AP and CMV/SEAP were concentrated in an Amicon ultrafiltration cell, followed by serial dilutions in HBHA buffer (Hank's balanced salt solution with 0.5 mg/ml BSA, 0.1% NaN₃, 20 mM HEPES (pH 7.0)). After equilibrium incubation with the ligand dilution series, cells were washed extensively in HBHA, lysed, and assayed calorimetrically for bound AP activity as described above. Bound and total PrP-AP was determined for K_{d} affinity studies; Bₘₐₓ determination (number of binding sites per cells) was calculated from the specific activity of AP (1 pmol of PrP-AP corresponds to approximately 3 OD units under the conditions and incubation periods of the assay). Specific binding of purified ³⁵[S]-labeled PrP-AP may be readily calculated by competition with unlabeled PrP-AP and Bmax values can be calculated from the X-intercept of the ³⁵[S]-labeled PrP-AP competition studies.) The computer program LIGAND was used to plot and analyze Scatchard data. G8 cells were found to possess ~1 X 10⁵ PrP-AP binding sites, with a dissociation constant (K_{d}) of 1.48 X 10⁻⁹ M. This high affinity binding is consistent with a specific receptor-ligand interaction.

To determine whether the conformation of the PrP was important for high affinity binding to PrPBPs, ³⁵[S]-labeled PrP-AP was boiled at 100°C for 5 minutes to disrupt its conformation. This treatment completely ablated binding to NIH 3T3 cells, indicating that the conformation of the PrP protein is important for high affinity binding to PrPBPs.

### Binding of PrP with PrPBPs transduce physiological signals such as those affecting cell proliferation and viability

To determine the physiological function of PrPBPs, the G8 muscle cell line known to possess PrPBPs was incubated with either PrP-AP or AP proteins, and proliferation of cells measured. Sister G8 cultures in 24 well plates were incubated for 3 days in COS cell supernatants containing PrP-AP or AP 25% v/v. Cells were counted in culture wells in eight high power fields after a three day incubation with the two expressed proteins. Results indicated that spontaneous proliferation of the G8 myoblast cell line was suppressed in the presence of PrP-AP (64 ± 9.8 SEM cells) compared to AP alone (283 ± 24.5 cells; p=0.002, Mann-Whitney nonparametic test).

Qualitatively, G8 cell cultures exposed to PrP-AP appeared to have more small, phase-dark, unattached cells than AP exposed cultures, suggesting that decreased proliferation may be due to increased cell death (Figure 6). The reduced cell number observed in G8 cell cultures exposed to PrP-AP strongly suggests that binding of PrP to PrPBPs transduces a signal which inhibits cell proliferation or promotes cell death.

### Characterization of PrPBPs

To verify that the PrPBPs described above were indeed proteins, sister NIH 3T3 cell cultures were incubated in calcium-free HBSS in the presence or absence of 0.25% trypsin for 30 minutes at room temperature, followed by PrP-AP incubation and calorimetric assessment of bound AP activity as described previously. This relatively mild proteolysis was sufficient to completely ablate surface PrP-AP binding to background levels (p < 0.001 by paired t-test). These results were consistent with the notion that PrPBPs are indeed proteins, or at least present on a protein backbone.

### Library Preparation

Two pcDNA3.1 plasmid libraries were prepared from C8 cells as follows. Total RNA was extracted with TRIzol (GIBCO BRL) and polyA + mRNA was isolated using Oligotex dT (Qiagen). Synthesis of double-stranded cDNA was carried out using the Time Saver cDNA Kit (Pharmacia) with either dT or random hexamer primers. The cDNA was then blunted with T4 DNA polymerase, adapted with Bam HI oligos, and size fractioned on an S-500 column (Pharmacia). Size selected cDNAs were then ligated into pcDNAa and the libraries electroporated into high efficiency competent E. coli (Invitrogen) by standard techniques.

### Plasmid Preparation, in vitro Transcription, and Microinjection

Both pcDNA3.1 plasmid libraries comprised approximately 0.5 X 10⁶ independent clones. Assuming that the mRNA abundance of the PrP ligand was "low moderate": (in the range of 0.01% of the transcripts), and that full-length cDNAs in the proper orientation would be successfully cloned in pCDNA3 at approximately 1/10 the frequency of corresponding mRNAs in the cell, we conservatively estimated that 1-5 X 10⁵ clones would have to be examined to identify a singly positive clone. The libraries were amplified in E. coli to yield pools of 1,500 to 2,000 colonies each. Individual pools were replica plated; one filter was used to grow and prepare plasmid DNA, and the other was stored. Plasmids from pools were prepared with plasmid MIDI kits (Qiagen), and mRNA was in vitro transcribed and capped from pools using mMessage mMachine (Ambion, Austin TX). *Xenopus* oocytes were prepared as previously described (Séguéla, P. et al., J. Neurosci. 16:448-455, 1996) and microinjected with 50 nL per oocyte (75-125 ng mRNA) with a nanoliter injector (World Precision Instruments).

### Cloning and Characterization of PrP-BPs

We estimated that 10-20 oocytes possess the membrane area of 500,000 G8 cells, upon which PrP-AP binding can be readily detected. Oocytes were monitored for PrPBP expression at 48 hours post injection by incubating with PrP-AP supernatant for 4 hours at room temperature. Eggs were then washed six times in HBHA. Uniform heating to inactivate endogenous cellular phosphatases was carried out in a heating block at 65°C. Bound AP activities were detected by incubation with 0.33 mg/ml NBT and 0.17 mg/ml BCIP in AP buffer (100 mM Tris-HCl, pH 9.5, 100 mM NaCl, 5 mM MgCl₂) for 0.5 to 12 hours.

In all series of injections, negative controls comprised of eggs injected with vector (pcDNA3.1, Invitrogen) alone and positive controls comprised of eggs injected with mRNA prepared from a ELF-1 plasmid clone (Chang et al., 79:157-168, 1994) and detected using a MEK-4-AP fusion protein were included.

After screening 33 pools (comprising approximately 660 individual Xenopus oocytes) which did not display any significant PrP binding, the 34th pool was found to be positive for PrP-AP binding. Subsequent pools were also negative for PrP binding. Pool 34 was fractionated by dilution of appropriate stock bacterial culture, into ten pools containing approximately 200 cDNA clones each. Of these, the pools demonstrating the highest level of PrP binding were chosen for further fractionation until single positive clones were isolated. Of the 32 individual cDNA clones tested for binding activity, clone 6 exhibited the highest level of PrP-AP binding activity over background. Clone 7, exhibited moderate PrP-AP binding.

### Sequencing

The cDNA inserts in clones 6 and 7 were sequenced using standard methods.

To search for homologies, the most recent updates of the EMBL and GENBANK nucleic acid databases were exploited using the BLAST network server (National Library of Medicine), and protein databases were examined using both the BLAST and the BLITZ network server (Heidelberg).

The cDNA insert in clone 6 encoded a portion of protocadherin-43 (amino acids 67-252 of protocadherin-43). The nucleic acid and deduced amino acid sequence of this portion of clone 6 is shown in Figures 7A and 7B. Protocadherin-43 is described by Sano et al. (*EMBO J.* 12:2249-2256, 1993). Protocadherin-43 sequence information is available: GENBANK L11373 (protocadherin-43).

The cDNA insert in clone 7 encoded a portion of OB-cadherin-1 (the amino terminal cadherin repeat). OB-cadherin-1 is described by Okazaki et al. (*J. Biol. Chem.* 269:12092-12098, 1994). OB-cadherin-1 sequence information is available: DBJ D21253 (mouse OB-cadherin) and SwissProt P55287 (human OB-cadherin).

Once a clone satisfying primary and secondary screening criteria is identified, a series of experiments are performed. For example, Northern blot analysis is carried out to reveal the size of the hybridizing mRNA species and also to determine whether there are multiple related transcripts of differing sizes. Northern blots of different cells, organs, and species, as well as blots using tissues from different developmental and disease states (particularly mouse scrapie) provide important information regarding cell specificity and regulation of the PrPBP. Discrepancies between cell surface PrP-AP binding and PrPBP expression would suggest that some cells express the PrPBP as a soluble species. In addition, using the amino acid sequences, immunogenic sequences are identified which facilitate the production of useful antibody probes against the PrPBP. Identification of the sequence for a physiologically appropriate PrPBP also permits numerous studies which further advance the understanding of prion disease and other neurodegenerative diseases, and which enable the development of small molecule therapeutics to block interaction or inactivate this transducing molecule as a treatment of these diseases.

In the alternative, PrPBPs may be cloned by purification of the PrPBP protein using a PrP-AP affinity column and standard column purification techniques. Useful sources for PrPBPs include tissue, cell, or membrane homogenates. Following column isolation, the PrPBP is characterized biochemically, yielding information about such properties as molecular weight, pK, and glycosylation. In addition, the protein is microsequenced, facilitating database searches and cDNA cloning using degenerate oligonucleotides and standard techniques of hybridization screening or PCR amplification.

### The Cadherins

The cadherins were initially recognized as calcium-dependent, homophilic cell-cell adhesion molecules, collaborating with members of other adhesion molecule families (including the integrins, immunoglobulin family adhesion molecules, and the selectins) in cell-cell recognition and adhesion phenomena in developmental formation and maintenance of tissues.

The cadherin superfamily comprises a diverse group of proteins defined by possession of extracellular "cadherin motifs" (having approximately 110 amino acids), which fold into repeated domains sharing some tertiary structural features of the immunoglobulin fold. By contrast, the cytoplasmic domains of the cadherins markedly diverge, reflecting in part the widely differing functions of these molecules. Sequence homology analysis suggests that the cadherin superfamily can be considered as at least two sub-families: the classical cadherins and the protocadherins. Others view the cadherins as falling into four functional subgroups: the classical cadherins, the desmosomal cadherins, the protocadherins, and other cadherin-related proteins (Suzuki, *J. Cell*. *Biochem.* 61:531-542, 1996).

The first recognized cadherin was E-cadherin, or uvomorulin, found to mediate calcium-dependent compaction of the blastomere in early development. The original classical cadherins were all homophilic adhesion molecules with five extracellular cadherin domains. This grouping also includes M-cadherin, N-cadherin, P-cadherin, and R-cadherin. More recently discovered classical cadherins (which may constitute a subfamily) include cadherins 5-13. Several of these new atypical "classical" cadherins (including cadherin 5 and 8) do not seem to confer homophilic binding activity when transfected into L cells, although they exhibit calcium-dependent localization at points of cell-cell contact, suggesting the existence of heterophilic ligands (Suzuki, *J. Cell. Biochem.* 61:531-542, 1996). Even classical cadherins demonstrating homophilic binding may also possess heterophilic binding ligands as suggested by integrin αEβ7 binding to E-cadherin (Cepek, *Nature* 372:190-193, 1994) and fibroblast growth factor binding to N-cadherin.

It is likely that many heterophilic ligands of the cadherins have yet to be identified. We have demonstrated using the PrP-AP fusion protein as a detection reagent and the frog oocyte expression system that prion proteins act as novel ligands for cadherin proteins.

The adhesive and specificity-determining site of the classical cadherins is said to be contained in the most N-terminal cadherin domain, EC1 (Shapiro et al., *Nature* 374:327-337, 1995). The C-terminal end of the cytoplasmic domain of most of the classical cadherins is well conserved, reflecting their binding to catenins and hence the cellular cytoskeleton (Ozawa et al., 1990, Hirano et al., 1992). Exceptions to the rule are T-cadherin (cadherin-13) and a splice variant of cadherin 8. Cadherin 13 does not possess a transmembrane domain, but similar to the prion protein, is anchored to the cell surface via a glycosyl-phosphatidylinositol tail, and cadherin 8 may be secreted by cells as a soluble isoform, completely lacking membrane anchorage of any type (Suzuki et al., 1996).

The protocadherins are a large, incompletely characterized group of proteins with homology to classical cadherins through possession of extracellular cadherin domains. However, the characterized protocadherins all appear to possess more than 5 cadherin domains, with similarity most marked to the EC3 and EC5 domains of the classical cadherins (Sano et al., *EMBO J.* 12:2249-2256, 1993). Thus, the EC1 binding motif of the classical cadherins is not shared with protocadherins; as a corollary, homophilic adhesive activity of protocadherins is not shared by all known members of this family of proteins.

Protocadherins also possess cytoplasmic domains which diverge from those of the classical cadherins, and from each other, suggesting specialized functional roles of protocadherins. It has therefore been postulated that protocadherins bind to novel and currently unknown heterophilic molecules.

The functions of the cadherins are not completely known at present. Clearly, so me classical cadherins participate in cell-layer segregation and morphogenesis in development. N-cadherin plays an important role in axonal extension. Cadherins also participate in maintenance of cell-cell recognition in mature tissues, and may participate in disorders in which recognition is deficient, such as metastatic cancer. A role in tumorigenesis is suggested by the competition of the wnt proto-oncogene product for beta-catenin. Pemphigus is an autoimmune disease mediated by immune recognition of desmosomal cadherins. It is also possible that the cadherins also participate in neurodegenerative disease, muscle disease, and immunological disease. The involvement of prion proteins in binding to cadherins will lead many to develop agents which disrupt or enhance the actions of prion proteins on cadherin function or the action of cadherins on prion function.

### PrPBP Binding to PrP is Calcium Dependent

Classical cadherins were first recognized by virtue of participation in homophilic cell-cell adhesion reactions which were calcium dependent. Further work has shown that calcium is not required for the adhesive interaction itself, but reacts with calcium binding sites between the cadherin folds to make the molecule more rigid and stable on the cell surface. (Shapiro et al., Nature 374:327-337, 1995). Even cadherin family members which do not participate in homophilic binding interactions can be induced by calcium to be localized at points of cell-cell contact (suggesting calcium-mediated binding to a heterophilic ligand).

In order to further investigate whether the PrPBPs originally identified on the surface of G8 and COS cells possessed the main feature of cadherin proteins--calcium-dependent binding--the following experiments were performed. PrP-AP supernatant was incubated with 2 mM EDTA for 2 hrs at room temperature to chelate the calcium in the media. Concurrently, confluent COS and G8 cells were incubated in PBS containing 1mM EDTA for 5 minutes to lift cells from bottom of the plate. Cells were then washed once in DMEM 5% FCS, and control cells incubated in HBHA containing 1.3 mM CaCl₂ and 1 mM MgCl₂ only, while experimental cells were incubated in PBS containing 1mM EDTA at room temperature for 30 minutes to remove calcium in the media. The first group of negative control cells were pelleted and resuspended in DMEM 5% (devoid of PrP-AP). The second group of positive control cells (calcium present in medium) and a third group of experimental cells (treated with EDTA) were resuspended in EDTA-treated PrP-AP for 1.5 hours at room temperature, followed by three washes in cold HBHA. The cells were then lysed as previously described and their supernatants examined for PrP-AP activity.

Binding of PrP-AP to both COS and G8 cells was significantly reduced in the presence of the calcium chelator EDTA (p<0.0001 for both COS a nd G8 cells, N=6) indicating the requirement for the presence of divalent cations such as Ca²⁺ for optimum binding.

### PrPBP Binding to PrP Displays Calcium Dependent Trypsin Resistance

Calcium binding protects some cadherins from trypsin digestion (Takeichi, *J. Cell. Biol*. 75:464:474, 1977), probably by inducing a conformational change which masks protease-sensitive sites.

In order to investigate whether the PrPBP originally identified on the surface of G8 and COS cells possessed a main feature of cadherin proteins--resistance to proteolysis in the presence of calcium--the following experiments were performed. Confluent COS and G8 cells were incubated in PBS containing 0.25% trypsin and 10 mM CaCl₂ for 20 minutes at 37°C or PBS containing 0.25% trypsin and 1 mM EDTA and then washed three times in DMEM 5% FCS. Approximately 1 x 10⁶ cells were distributed per eppendorf tube and these were washed twice with HBHA media. The cells were then incubated with PrP-AP supernatant for 1.5 hours at room temperature, followed by three washes in cold HBHA. The cells were then lysed on ice in 50 mM Tris, 150 mM NaCl, 1% Triton-X100®, 0.02% NaN₃ pH 8.0. The lysate was microcentrifuged and the supernatant was uniformly heated on a heating block to 65°C for 12 minutes to inactivate endogenous cellular phosphatases. Bound AP activity was detected by incubation with NBT and BCIP in AP buffer (100 mM Tris-HCl, pH 9.5,10 0 mM NaCl, 5 mM MgCl₂ for 0.5 to 12 hours).

In the presence of Ca²⁺ ions PrP-AP binding to COS and G8 cells was maintained, despite treatment of the cells with trypsin prior to their incubation with PrP-AP. In the absence of Ca²⁺, there was a significant reduction of PrP-AP binding (p<0.001 for COS and p<0.05 for G8 cells, N=4). This data indicates that the PrPBP species on the surface of the COS and G8 cells is protected by trypsin proteolysis in the presence of Ca²⁺, a known characteristic of cadherin molecules (Takahashi et al., *Oncogene* 8:2925-2929, 1993). This data therefore supports the notion that PrPs bind to cadherin family members.

### Isolation and cloning of a human PrPBP

Human PrPBP may be isolated as described above for the murine homolog. In particular, a recombinant human PrP-AP fusion gene is constructed essentially as described above, using the human PrP cDNA described by Kretzschmar et al. (DNA 5:315-324, 1986) in place of the murine PrP gene. The human PrP fragment is generated by standard PCR amplification using the following primers: and This fragment is then cloned in-frame into the APtag-2 expression vector (Cheng and Flanagan, supra). Using the fusion protein product expressed from this vector, human PrPBPs are isolated by any of the affinity techniques described above (for example, by precipitation from human PrPBP-producing cells). Verification that the protein isolated is, in fact, a human PrPBP may also be accomplished as described above.

Once isolated, the human PrPBP protein may be microsequenced, and the amino acid sequence used to design oligonucleotides for hybridization screening or PCR primers for amplification of the human PrPBP coding sequence from any appropriate human cDNA or genomic DNA library.

Alternatively, the human PrP-AP fusion protein is used to isolate a human PrPBP-expressing cDNA clone using the pcDNA3.1 cloning system and the techniques described above. Or, preferably, in yet another alternative technique, a reduced stringency screening of a human brain cDNA library is performed using the mouse PrPBP coding sequence (described above) as a probe. Again, mouse PrPBP sequences may be used as hybridization probes, or PCR primers may be designed based upon regions of the mouse PrPBP sequence likely to be conserved across different species and used for amplification of the human sequence.

### PrPBP Protein Expression

In general, PrPBPs used in the invention may be produced by transformation of a suitable host cell with all or part of a PrPBP-encoding cDNA fragment in a suitable expression vehicle.

Those skilled in the field of molecular biology will understand that any of a wide variety of 'expression systems may be used to provide the recombinant protein. The precise host cell used is not critical.

The PrPBP may be produced in a prokaryotic host (e.g., E.coli) or in a eukaryotic host (e.g., Saccharomyces cerevisiae, insect cells, e.g., Sf21 cells, or mammalian cells, e.g., NIH 3T3, HeLa, or preferably COS cells). Such cells are available from a wide range of sources (e.g., the American Type Culture Collection, Rockland, MD; also, see, e.g., Ausubel et al., Current Protocols in Molecular Biology, John Wiley & Sons, New York, 1994). The method of transformation or transfection and the choice of expression vehicle will depend on the host system selected. Transformation and transfection methods are described, e.g., in Ausubel et al. (supra); expression vehicles may be chosen from those provided, e.g., in Cloning Vectors: A Laboratory Manual (P.H. Pouwels et al., 1985, Supp. 1987).

Alternatively, a PrPBP may be produced by a stably-transfected mammalian cell line. A number of vectors suitable for stable transfection of mammalian cells are available to the public, e.g., see Pouwels et al. (supra); methods for constructing such cell lines are also publicly available, e.g., in Ausubel et al. (supra). In one example, cDNA encoding the PrPBP is cloned into an expression vector which includes the dihydrofolate reductase (DHFR) gene. Integration of the plasmid and, therefore, the PrPBP-encoding gene into the host cell chromosome is selected for by inclusion of 0.01-300 µM methotrexate in the cell culture medium (as described in Ausubel et al., supra). This dominant selection can be accomplished in most cell types. Recombinant protein expression can be increased by DHFR-mediated amplification of the transfected gene. Methods for selecting cell lines bearing gene amplifications are described in Ausubel et al. (supra); such methods generally involve extended culture in medium containing gradually increasing levels of methotrexate. DHFR-containing expression vectors commonly used for this purpose include pCVSEII-DHFR and pAdD26SV(A) (described in Ausubel et al., supra). Any of the host cells described above or, preferably, a DHFR-deficient CHO cell line (e.g., CHO DHFR⁻ cells, ATCC Accession No. CRL 9096) are among the host cells preferred for DHFR selection of a stably-transfected cell line or DHFR-mediated gene amplification.

Once the recombinant PrPBP is expressed, it is isolated, e.g., using affinity chromatography. In one example, an anti-PrPBP antibody (e.g., produced as described herein) may be attached to a column and used to isolate the PrPBP. Lysis and fractionation of PrPBP-harboring cells prior to affinity chromatography may be performed by standard methods (see, e.g., Ausubel et al., supra).

Once isolated, the recombinant protein can, if desired, be further purified, e.g., by high performance liquid chromatography (see, e.g., Fisher, Laboratory Techniques In Biochemistry And Molecular Biology, eds., Work and Burdon, Elsevier, 1980).

Polypeptides particularly short PrPBP fragments, can also be produced by chemical synthesis (e.g., by the methods described in Solid Phase Peptide Synthesis, 2nd ed., 1984 The Pierce Chemical Co., Rockford, IL).

These general techniques of polypeptide expression and purification can also be used to produce and isolate useful PrPBP fragments or analogs (described herein).

### Anti-PrPBP Antibodies

To generate PrPBP-specific antibodies, a PrPBP coding sequence may be expressed as a C-terminal fusion with glutathione S-transferase (GST) (Smith et al., Gene 67:31-40, 1988). The fusion protein is purified on glutathione-Sepharose beads, eluted with glutathione, cleaved with thrombin (at the engineered cleavage site), and purified to the degree necessary for immunization of rabbits. Primary immunizations are carried out with Freund's complete adjuvant and subsequent immunizations with Freund's incomplete adjuvant. Antibody titres are monitored by Western blot and immunoprecipitation analyses using the thrombin-cleaved PrPBP protein fragment of the GST-PrPBP fusion protein. Immune sera are affinity purified using CNBr-Sepharose-coupled PrPBP protein. Antiserum specificity is determined using a panel of unrelated GST proteins.

As an alternate or adjunct immunogen to GST fusion proteins, peptides corresponding to relatively unique immunogenic regions of PrPBP may be generated and coupled to keyhole limpet hemocyanin (KLH) through an introduced C-terminal lysine. Antiserum to each of these peptides is similarly affinity purified on peptides conjugated to BSA, and specificity tested in ELISA and Western blots using peptide conjugates, and by Western blot and immunoprecipitation using PrPBP expressed as a GST fusion protein.

Alternatively, monoclonal antibodies may be prepared using the PrPBPs described above and standard hybridoma technology (see, e.g., Kohler et al., Nature 256:495, 1975; Kohler et al., Eur. J. Immunol. 6:511, 1976; Kohler et al., Eur. J. Immunol. 6:292, 1976; Hammerling et al., In Monoclonal Antibodies and T Cell Hybridomas, Elsevier, NY, 1981; Ausubel et al., supra). Once produced, monoclonal antibodies are also tested for specific PrPBP recognition by Western blot or immunoprecipitation analysis (by the methods described in Ausubel et al., supra). Antibodies which specifically recognize PrPBP are considered to be useful; such antibodies may be used, e.g., in an immunoassay to monitor the level of PrPBP produced by a mammal (for example, to determine the amount or subcellular location of PrPBP). Alternatively monoclonal antibodies may be prepared using the PcPBP described above and a phage display library (Vaughan et al., Nature Biotech 14:309-314, 1996).

Preferably, antibodies are produced using fragments of the PrPBP which lie outside generally conserved regions and appear likely to be antigenic, by criteria such as high frequency of charged residues. In one specific example, such fragments are generated by standard techniques of PCR and cloned into the pGEX expression vector (Ausubel et al., supra). Fusion proteins are expressed in E. coli and purified using a glutathione agarose affinity matrix as described in Ausubel et al. (supra). To attempt to minimize the potential problems of low affinity or specificity of antisera, two or three such fusions are generated for each protein, and each fusion is injected into at least two rabbits. Antisera are raised by injections in a series, preferably including at least three booster injections.

### Prion Disease Detection Assays

PrPBPs find diagnostic use generally in the detection or monitoring of prion diseases. For example, PrPBPs may be used to monitor the presence or absence of PrP^{sc} in a biological sample (e.g., a tissue biopsy or fluid) using standard detection assays. Such detection assays include, but are not limited to, those that rely on detectably-labeled components, for example, a labeled PrPBP, a labeled PrPBP fusion protein, or a labelled PrP^{sc}. Assays may involve direct detection of a PrP^{sc} or may involve indirect detection (for example, by binding PrPBP to a PrP and then detecting the complex using a second labeled antibody directed against PrPBP). PrPBPs (for example, mouse or human PrPBPs) for use in these detection assays may be produced recombinantly in any cell, but COS cells are a preferred host cell (e.g., according to the methods of Cullen, Meth. Enzymol. 152:684-704, 1988). Alternatively, the PrPBP may be isolated by affinity purification using a PrP-AP protein and standard techniques. In one particular preferred embodiment, soluble PrPBP binding domains are expressed and used in detection methods. In another preferred embodiment, PrPBP binding domains are expressed as fusion proteins containing both mouse and human PrPBP coding sequences. Again, these fusion proteins are used in the detection methods described herein.

In one working example, a biological sample known or suspected to contain a prion agent is tested as follows. The biological sample is treated with a protease according to standard techniques (e.g., as described in Prusiner et al., Science 216:136-144, 1982). Following protease inactivation, the sample is incubated with either a detectably labeled PrPBP (or a fragment or analog thereof) or PrPBP fusion protein present in a known concentration, and the resulting complex isolated or identified, for example, by filter binding, immunoprecipitation, gel electrophoretic resolution, sedimentation, or filtration. The isolated complex is then analyzed and measured according to standard methods. Any appropriate label which may be directly or indirectly visualized may be utilized in these detection assays including, without limitation, any radioactive, fluorescent, chromogenic (e.g., alkaline phosphatase or horseradish peroxidase), or chemiluminescent label, or a hapten (for example, digoxigenin or biotin) which may be visualized using a labeled, hapten-specific antibody or other binding partner (e.g., avidin). If immunological-based assays are performed, polyclonal or monoclonal antibodies produced against PrPBP (as described above) or PrP (Bendheim et al., Nature 310:418-21, 1984) may be used in any standard immunoassay format (e.g., ELISA, Western blot, or RIA assay) to measure complex formation. Examples of such immunoassays are described, e.g., in Ausubel et al., supra. Samples found to contain increased levels of labeled complex compared to appropriate control samples are taken as indicating the presence of a prion protein, and are thus indicative of a prion-related disease.

In another detection assay, PrP^{sc} in a biological sample is captured on a solid support (e.g., nitrocellulose, Immobilon, agarose beads, cyanogen-activated agarose, or tissue culture wells), and binding by PrPBP (or a fragment or analog thereof) or PrPBP fusion protein is detected. In the alternative, a PrPBP or a PrPBP fusion protein is bound to a solid support, and binding of PrP^{sc} is detected. In these assay formats, detection is accomplished by any standard method as described above, for example, detection of PrP^{sc} in association with a solid support is accomplished directly or indirectly using PrPBP as described above. Alternatively, PrP^{sc} in a captured PrP^{sc}:PrPBP complex may be detected using antibodies directed against PrP^{sc} (Bendheim et al., Nature 310:418-21, 1984). Again, any appropriate label may be utilized, for example, any radioactive or fluorescent marker may be used or, alternatively, any enzyme marker may be utilized, including (without limitation) alkaline phosphatase or horseradish peroxidase, and detection may be accomplished by addition of a chromogenic or fluorogenic substrate.

As noted above, a PrPBP which bind PrP^{c}, but not PcP^{sc} can be used to remove PrP^{c} from a biological sample. The remaining PcP^{sc} in the sample can be quatitated by binding to a reagent, e.g., an antibody which binds both PrP^{sc} and PrP^{c}. Thus, even a PrPBP which binds PrP^{c}, but does not bind PrP^{sc} can be used in an assay for PrP^{sc}.

In another detection assay, a detectably-labelled PrP^{sc} is incubated with PrPBP, followed by incubation with a biological sample suspected of containing PrP^{sc} (if desired, following protease treatment). The resulting complexes are then measured and quantified according to conventional methods as described above. In this assay format, a decrease in the presence of labeled PrP^{sc} found in the PrPBP:PrP^{sc} complex as compared to control samples is taken as an indication that PrP^{sc} is present in the biological sample, since unlabeled PrP^{sc} found in the sample competitively displaces the labeled PrP^{sc} present in the complex.

### Prion Decontamination

The methods and compositions described herein are useful for the decontamination of biological samples that are known or suspected of being contaminated with a prion protein. In particular, because PrPBP is capable of binding to PrP^{sc}, biological samples may be incubated with PrPBP, and the PrPBP complexes removed using standard methods, e.g., secondary sequestration or separation methodologies. Alternatively, PrPBPs may simply be incubated with biological samples to complex with, and thereby inhibit, the effects of PrP.

### Treatment of Prion-Related Diseases and Other Neurodegenerative Diseases

The disclosed methods and compositions also provide a means for treating or preventing prion diseases in mammals, including humans. This treatment may be accomplished directly, e.g., by treating the animal with antagonists which disrupt, suppress, attenuate, or neutralize the biological events associated with PrPBP:PrP^{sc} complex formation. Such antagonists may be isolated using PrPBPs and the functional assays described above. For example, the invention provides a means for the identification of compounds (e.g., peptides, small molecule inhibitors, or mimetics) that are capable of antagonizing PrPBP:PrP^{sc} complex interactions. Such antagonists include, without limitation, PrPBP, PrPBP fragments or analogs that bind and inactivate the biological activity of PrP^{sc}, anti-PrPBP antibodies, fragments or analogs of PrP PrP^{sc} or PrP^{c} that interact with PrPBP and block PrPBP:PrP complex formation and disrupt the associated biological activity resulting from such complex formation; or peptide or non-peptide molecules, including pharmacological agents, that interfere and block the biological activity associated with PrP^{sc}, PrPBP, or PrPBP:PrP^{sc} complex formation, and that are found, e.g., in a cell extract, mammalian serum, or growth medium in which mammalian cells have been cultured.

Antagonist molecules include: molecules selected for their ability to bind with high affinity to PrP^{sc} and render it incapable of recruiting more copies of itself from PrP^{c}; molecules capable of binding with high affinity to PrP^{c} and block its conversion to PrP^{sc}; molecules capable of blocking the generation of cytotoxic signals by preventing the interaction of PrP^{c} with PrP^{sc} or complexes thereof; molecules capable of blocking the generation of soluble toxic mediators (e.g., reactive oxygen species and expression of tumor necrosis factor) by neighboring cells expressing PrP^{c}; or any combination of these aforementioned mechanisms.

The efficacy of a candidate antagonist, e.g., PrPBP, is dependent upon its ability to interact with a prion protein. Such an interaction can be readily assayed using any number of standard binding techniques and functional assays (e.g., those described herein). In one particular example, candidate antagonists may be tested in vitro for interaction with PrP^{sc} and their ability to modulate PrP^{sc}-mediated biological activity may be assayed by any of the functional tests described herein.

A molecule that promotes a decrease in PrPBP:PrP^{sc} complex formation or a decrease in PrPBP:PrP^{sc}-mediated biological activity in vitro is considered useful for therapy; such a molecule can be used, for example, as a therapeutic to treat or prevent the onset of a prion disease.

Evaluation of whether a test antagonist confers protection against the development of a prion disease in vivo generally involves using an animal known to develop such a disease (e.g., Chandler, Lancet 6:1378-1379, 1961; Eklund et al., J. Infectious Disease 117:15-22, 1967; Field, Brit. J. Exp. Path. 8:129-239, 1969). An appropriate animal (for example, a mouse or hamster) is treated with the test compound according to standard methods, and a reduced incidence or delayed onset of a prion-related illness, compared to untreated control animals, is detected as an indication of protection. The test compound may be administered to an animal which has previously been injected with a prion agent or, alternatively, the test compound may be tested for its ability to neutralize a prion agent by pre-incubating the prion and the compound and injecting the prion/compound mixture into the test animal. A molecule (e.g., an antagonist as described above) that is used to treat or prevent a prion disease is referred to as an "anti-prion therapeutic."

An anti-prion therapeutic may be administered with a pharmaceutically-acceptable diluent, carrier, or excipient, in unit dosage form. For example, conventional pharmaceutical practice may be employed to provide suitable formulations or compositions to administer such anti-prion therapeutics to animals suffering from or presymptomatic for a prion disease, or at risk for developing a prion disease. Any appropriate route of administration can be employed, for example, parenteral, intravenous, subcutaneous, intramuscular, intracranial, intraorbital, ophthalmic, intraventricular, intracapsular, intraspinal, intracisternal, intraperitoneal, intranasal, aerosol, or oral administration.

Methods well known in the art for making formulations are found in, for example, "Remington's Pharmaceutical Sciences." Formulations for parenteral administration can, for example, contain excipients, sterile water, or saline, polyalkylene glycols such as polyethylene glycol, oils of vegetable origin, or hydrogenated napthalenes. Biocompatible, biodegradable lactide polymer, lactide/glycolide copolymer, or polyoxyethylene-polyoxypropylene copolymers can be used to control the release of the compounds. Other potentially useful parenteral delivery systems for anti-prion therapeutic compounds include ethylene-vinyl acetate copolymer particles, osmotic pumps, implantable infusion systems, and liposomes. Formulations for inhalation can contain excipients, for example, lactose, or can be aqueous solutions containing, for example, polyoxyethylene-9-lauryl ether, glycocholate and deoxycholate, or can be oily solutions for administration in the form of nasal drops, or as a gel.

The methods disclosed herein may be used to reduce or prevent the disorders described herein in any animal, for example, humans, domestic pets, or livestock. Where a non-human animal is treated, the anti-prion therapeutic employed is preferably specific for that species.

## Claims

1. A method for identifying a nucleic acid molecule which encodes a prion binding protein, wherein said prion binding protein binds to a prion protein under physiological conditions and in a conformation-dependent manner, said method comprising:
a) providing a population of frog oocytes transfected with a pool of mammalian cDNA molecules;
b) exposing the population frog oocytes to detectably labelled PrP;
c) identifying a frog oocyte which binds detectably labelled PrP;
d) providing a second population of frog oocytes transfected with cDNA molecules identical to those present in the oocyte selected in step c);
e) exposing the second population of frog oocytes to detectably labelled PrP; and
f) identifying a frog oocyte in the second population which binds detectably labelled PrP;
g) obtaining cDNA molecule identical to that used to transfect the frog oocyte identified in step f), thereby identifying a nucleic acid molecule which encodes a prion binding protein that binds to prion protein under physiological conditions and in a conformation-dependent matter.

2. The method of claim 1, wherein said detectably labelled PrP binds to said prion binding protein with high affinity.

3. The method of claim 1, wherein said detectably labelled PrP exhibits conformation dependent binding to said prion binding protein.

4. The method of claim 1, wherein said prion binding protein is from a human, a domesticated farm animal, or a pet species.

5. The method of claim 1, wherein said prion binding protein is from a mammal.

6. The method of claim 1, wherein said mammal is a cow, sheep, or goat.

7. A method for removing PrP^{sc} from a biological sample, comprising
a) contacting the biological sample with a PrPBP which is encoded by a nucleic acid molecule identifiable by the method of claim 1 and binds to PrP under physiological conditions and in a conformation-dependent manner under conditions which allow the formation of complexes between PrP^{sc} and the PrPBP; and
b) recovering the PrP^{sc} : PrPBP complex from the biological sample.

8. The use of all or a PrP binding portion of a cadherin for the preparation of a pharmaceutical composition for treating prion diseases.

9. The use of claim 8, wherein the cadherin is protocadherin 43.

10. The use of claim 8, wherein the cadherin is OB-cadherin-1.

11. The use of claim 8, wherein the selected prion binding protein has the sequence of SEQ ID NO:2.

12. A method for identifying potential therapeutic compounds for the treatment of a disorder associated with the presence of prion protein, said method comprising:
a) measuring the binding of a selected prion binding protein to PrP^{sc} or PrP^{c} in the presence of a test compound, wherein said prion binding protein is encoded by a nucleic acid molecule identifiable by the method of claim 1 and binds to prion protein in a saturable and displaceable and conformation-dependent manner and under physiological conditions.
b) measuring the binding of the selected prion binding protein to PrP^{sc} of PrP^{c} in the absence of the test compound;
wherein binding of the selected prion binding protein to PrP^{sc} or PrP^{c} in the presence of the test compound that is less than binding of the selected prion binding protein to PrP^{sc} or PrP^{c} in the absence of the test compound indicates that the test compound is a potential therapeutic compound for the treatment of a disorder associated with the presence of prion protein.

13. The method of claim 12, wherein the selected prion binding protein is a cadherin.

14. The method of claim 12, wherein the cadherin is protocadherin 43.

15. The method of claim 12, wherein the cadherin is OB-cadherin-1.

16. The method of claim 12, wherein the selected prion binding protein has the sequence depicted in Fig. 7A+7B.

17. The method of claim 12, wherein said disorder associated with the presence of prion binding protein affects a human, a domesticated farm animal, or a pet species.

18. The method of claim 12, wherein said disorder associated with the presence of prion binding protein affects a human, cow, sheep, or goat.

## Patentansprüche

1. Ein Verfahren zur Identifizierung eines Nukleinsäuremoleküls, welches für ein Prion-bindendes Protein kodiert, wobei das Prion-bindende Protein an ein Prion-Protein unter physiologischen Bedingungen und in einer konformationsabhängigen Weise bindet, umfassend:
a) Bereitstellen einer Population von Froschoozyten, transfiziert mit einem Pool von Säuger-cDNA-Molekülen;
b) Inkubieren der Population von Froschoozyten mit nachweisbar markiertem PrP;
c) Identifizieren einer Froschoozyte, die nachweisbar markiertes PrP bindet;
d) Bereitstellen einer zweiten Population von Froschoozyten, die mit cDNA-Molekülen transfiziert sind, die identisch zu den cDNA-Molekülen sind, die in der in Schritt c) ausgewählten Oozyte anwesend sind;
e) Inkubieren der zweiten Population von Froschoozyten mit nachweisbar markiertem PrP; und
f) Identifizieren einer Froschoozyte in der zweiten Population, die nachweisbar markiertes PrP bindet;
g) Gewinnen eines cDNA-Moleküls, das zu dem cDNA-Molekül, welches zur Transfektion der in Schritt f) identifizierten Froschoozyte verwendet wurde, identisch ist, wobei ein Nukleinsäuremolekül identifiziert wird, das für ein Prion-bindendes Protein kodiert, welches ein Prion-Protein unter physiologischen Bindungen und in einer konformationsabhängigen Weise bindet.

2. Verfahren gemäß Anspruch 1, wobei das nachweisbar markierte PrP an das Prion-bindende Protein mit hoher Affinität bindet.

3. Verfahren gemäß Anspruch 1, wobei das nachweisbar markierte PrP konformationsabhängige Bindung an das Prion-bindende Protein aufweist.

4. Verfahren gemäß Anspruch 1, wobei das Prion-bindende Protein von einem Menschen, einem domestizierten Nutztier oder einer Haustierart stammt.

5. Verfahren gemäß Anspruch 1, wobei das Prion-bindende Protein von einem Säuger stammt.

6. Verfahren gemäß Anspruch 1, wobei der Säuger eine Kuh, ein Schaf oder eine Ziege ist.

7. Ein Verfahren zur Entfernung von PrP^{sc} aus einer biologischen Probe, umfassend
a) In-Kontakt-Bringen der biologischen Probe mit einem PrPBP, das durch ein Nukleinsäuremolekül, welches durch das Verfahren gemäß Anspruch 1 identifiziert werden kann, kodiert wird, und an PrP unter physiologischen Bedingungen und in einer konformationsabhängigen Weise unter Bedingungen bindet, die die Bildung von Komplexen zwischen PrP^{sc} und dem PrPBP erlauben; und
b) Gewinnen des PrP^{sc}:PrPBP-Komplexes aus der biologischen Probe.

8. Die Verwendung des vollständigen Cadherins oder eines PrP-bindenden Teils eines Cadherins zur Herstellung einer pharmazeutischen Zusammensetzung zur Behandlung von Prion-Erkrankungen.

9. Verwendung gemäß Anspruch 8, wobei das Cadherin Protocadherin 43 ist.

10. Verwendung gemäß Anspruch 8, wobei das Cadherin OB-Cadherin-1 ist.

11. Die Verwendung gemäß Anspruch 8, wobei das ausgewählte Prion-bindende Protein die Sequenz von SEQ ID NO:2 besitzt.

12. Ein Verfahren zur Identifizierung möglicher therapeutischer Verbindungen zur Behandlung einer Erkrankung, die mit der Anwesenheit von Prion-Protein assoziiert ist, umfassend:
a) Messen der Bindung eines ausgewählten Prion-bindenden Proteins an PrP^{sc} oder PrP^{c} in Anwesenheit einer Testverbindung, wobei das Prion-bindende Protein von einem Nukleinsäuremolekül kodiert wird, welches durch das Verfahren gemäß Anspruch 1 identifiziert werden kann, und an Prion-Protein in einer sättigenden und verdrängbaren und konformationsabhängigen Weise und unter physiologischen Bedingungen bindet;
b) Messen der Bindung des ausgewählten Prion-bindenden Proteins an PrP^{sc} oder PrP^{c} in Abwesenheit der Testverbindung;
wobei die Bindung des ausgewählten Prion-bindenden Proteins an PrP^{sc} oder PrP^{c} in Anwesenheit der Testverbindung, die geringer als die Bindung des ausgewählten Prion-bindenden Proteins an PrP^{sc} oder PrP^{c} in Abwesenheit der Testverbindung ist, anzeigt, dass die Testverbindung eine mögliche therapeutische Verbindung zur Behandlung einer Erkrankung darstellt, die mit der Anwesenheit von Prion-Protein assoziiert ist.

13. Verfahren gemäß Anspruch 12, wobei das ausgewählte Prion-bindende Protein ein Cadherin ist.

14. Verfahren gemäß Anspruch 12, wobei das Cadherin Protocadherin 43 ist.

15. Verfahren gemäß Anspruch 12, wobei das Cadherin OB-Cadherin-1 ist.

16. Verfahren gemäß Anspruch 12, wobei das ausgewählte Prion-bindende Protein die in den Fig. 7A und 7B dargestellte Sequenz aufweist.

17. Verfahren gemäß Anspruch 12, wobei die Erkrankung, die mit der Anwesenheit von Prion-bindendem Protein assoziiert ist, einen Menschen, ein domestiziertes Nutztier oder eine Haustierart beeinträchtigt.

18. Verfahren gemäß Anspruch 12, wobei die Erkrankung, die mit der Anwesenheit von Prion-bindendem Protein assoziiert ist, einen Menschen, Kuh, Schaf oder Ziege beeinträchtigt.

## Revendications

1. Procédé pour l'identification d'une molécule d'acide nucléique qui code pour une protéine de liaison au prion, dans lequel ladite protéine de liaison au prion se fixe à une protéine de prion dans les conditions physiologiques et d'une manière dépendante de la conformation, ledit procédé comprenant:
a) la fourniture d'une population d'oocytes de grenouille transfectés avec un pool de molécules d'ADNc mammaliennes;
b) l'exposition de la population d'oocytes de grenouille à une PrP marquée de manière détectable;
c) l'identification d'un oocyte de grenouille qui fixe la PrP marquée de manière détectable;
d) la fourniture d'une deuxième population d'oocytes de grenouille transfectés avec des molécules d'ADNc identiques à celles présentes dans l'oocyte choisi dans l'étape c);
e) l'exposition de la deuxième population d'oocytes de grenouille à de la PrP marquée de manière détectable; et
f) l'identification d'un oocyte de grenouille dans la deuxième population qui fixe la PrP marquée de manière détectable;
g) l'obtention de molécule d'ADNc identique à celle utilisée pour transfecter l'oocyte de grenouille identifié dans l'étape f), identifiant ainsi une molécule d'acide nucléique qui code pour une molécule de fixation au prion qui se lie à la protéine de prion dans les conditions physiologiques et d'une manière dépendante de la conformation.

2. Procédé selon la revendication 1, dans lequel ladite PrP marquée de manière détectable se fixe à ladite protéine de liaison au prion avec une forte affinité.

3. Procédé selon la revendication 1, dans lequel ladite PrP marquée de manière détectable présente une liaison dépendante de la conformation à ladite protéine de liaison au prion.

4. Procédé selon la revendication 1, dans lequel ladite protéine de liaison au prion provient d'un humain, d'un animal de ferme domestique, ou d'une espèce d'animal de compagnie.

5. Procédé selon la revendication 1, dans lequel la dite protéine de liaison au prion provient d'un mammifère.

6. Procédé selon la revendication 1, dans lequel ledit mammifère est une vache, un mouton ou une chèvre.

7. Procédé pour l'élimination de PrP^{sc} d'un échantillon biologique, comprenant
a) la mise en contact de l'échantillon biologique avec une PrPBP qui est codée par une molécule d'acide nucléique identifiable par le procédé selon la revendication 1 et se fixe à la PrP dans les conditions physiologiques et d'une manière dépendante de la conformation dans des conditions qui permettent la formation de complexes ente PrP^{sc} et la PrPBP ; et
b) le recouvrement du complexe PrP^{sc} : PrPBP de l'échantillon biologique.

8. Utilisation de la totalité ou d'une portion se liant à la PrP d'une cadhérine pour la préparation d'une composition pharmaceutique pour le traitement de maladies à prion.

9. Utilisation selon la revendication 8, dans laquelle la cadhérine est la protocadhérine 43.

10. Utilisation selon la revendication 8, dans laquelle la cadhérine est l'OB-cadhérine-1.

11. Utilisation selon la revendication 8, dans laquelle la protéine de liaison au prion choisie a la séquence de SEQ ID NO:2.

12. Procédé pour l'identification de composés thérapeutiques potentiels pour le traitement d'un trouble associé à la présence d'une protéine de prion, le dit procédé comprenant :
a) la mesure de la fixation d'une protéine de liaison au prion choisie à la PrP^{sc} ou PrP^{c} en présence d'un composé test, tandis que ladite protéine de liaison au prion est codée par une molécule d'acide nucléique identifiable par le procédé selon la revendication 1 et se fixe à la protéine de prion d'une manière saturable et déplaçable et dépendante de la conformation, et dans les conditions physiologiques;
b) la mesure de la fixation de la protéine de liaison au prion choisie à PrP^{sc} ou PTP^{c} en l'absence du composé test.
tandis que la fixation de la protéine de liaison au prion choisie à PrP^{sc} ou PrP^{c} en présence du composé test qui est inférieur à la fixation de la protéine de liaison au prion choisie à PrP^{sc} ou PrP^{c} en l'absence du composé test indique que le composé test est un composé thérapeutique potentiel pour le traitement d'un trouble associé à la présence de la protéine du prion.

13. Procédé selon la revendication 12, dans lequel la protéine de liaison au prion choisie est une cadhérine.

14. Procédé selon la revendication 12, dans lequel la cadhérine est la protocadhérine 43.

15. Procédé selon la revendication 12, dans lequel la cadhérine est l'OB-cadhérine-1.

16. Procédé selon la revendication 12, dans lequel la protéine de liaison au prion choisie a la séquence illustrée dans Fig. 7A + 7B.

17. Procédé selon la revendication 12, dans lequel ledit trouble associé à la présence de protéine de liaison au prion affecte un humain, un animal de ferme domestique, ou une espèce d'animal de compagnie.

18. Procédé selon la revendication 12, dans lequel ledit trouble associé à la présence de protéine de liaison au prion affecte un humain, une vache, un mouton ou une chèvre.
